# EUROPÄISCHE PATENTSCHRIFT

(11) **EP 2 446 247 B1**
(45) Veröffentlichungstag und Bekanntmachung des Hinweises auf die Patenterteilung: **01.05.2013**
(21) Anmeldenummer: 10730408.1
(22) Anmeldetag: 23.06.2010
(51) Int. Cl.: G01N 17/04, C07C 263/10, B01J 19/00

(54) **VERFAHREN ZUR ERFASSUNG VON WASSEREINTRITTEN IN PHOSGENFÜHRENDEN ANLAGEN**
METHOD FOR SENSING WATER INGRESS IN PHOSGENE-CONDUCTING SYSTEMS
PROCÉDÉ DE DÉTECTION D'ENTRÉES D'EAU DANS DES DISPOSITIFS CONDUISANT DU PHOSGÈNE

(30) Priorität: 24.06.2009 EP 09163666
(43) Veröffentlichungstag der Anmeldung: 02.05.2012
(73) Patentinhaber: BASF SE, 67056 Ludwigshafen (DE)
(72) Erfinder: SCHELLING, Heiner, 67281 Kirchheim (DE); PENZEL, Ulrich, 01945 Tettau (DE); STROEFER, Eckhard, 68163 Mannheim (DE); HABLAWETZ, Dirk, 67227 Frankenthal (DE); BECKWITH, Dave, Baton Rouge, Louisiana 5503 (US); THIELE, Kai, 2040 Antwerpen 4 (BE); JACOBS, Johannes, 4641 PE Ossendrecht (NL); EIERMANN, Matthias, 67117 Limburgerhof (DE); STORCK, Uwe, 67071 Ludwigshafen (DE); SPEIER, Jon S., Baton Rouge, Louisiana 70810 (US); GRZANKA, Thomas, Baton Rouge, Louisiana 70817 (US); PORTWOOD, William, Baton Rouge, Louisiana 13939 (US)
(86) Internationale Anmeldenummer: PCT/EP2010/058909
(87) Internationale Veröffentlichungsnummer: WO 2010/149701

(56) Entgegenhaltungen:
- DD-A3- 139 955
- DE-A1- 10 027 779
- JP-A- 8 261 914
- JP-A- 2008 128 783

## Beschreibung

Die Erfindung betrifft ein Verfahren zur Erfassung von Wassereintritten und dadurch resultierende Korrosion in Anlagen zur Herstellung von Isocyanaten durch Umsetzung von Phosgen mit einem oder mehreren primären Aminen in einem Lösungsmittel. Die Erfindung betrifft ferner eine Vorrichtung zur Herstellung solcher Isocyanate, in deren Aufarbeitungsteil an definierten Stellen Sonden zur Überwachung von Korrosion angeordnet sind.

Isocyanate und Isocyanatgemische können nach bekannten Verfahren durch Umsetzung primärer Amine oder deren Hydrochloride mit einem Überschuss an Phosgen hergestellt werden. Dabei werden die Edukte, das primäre Amin und das Phosgen, üblicherweise zusammen mit einem Lösungsmittel in eine Mischeinheit geleitet, die einem Reaktor vorgeschaltet ist. Nach dem Vermischen in der Mischeinheit wird das so erzeugte Gemisch in einen Reaktor überführt und zu dem entsprechenden Isocyanat umgesetzt. Nach dieser Umsetzung wird das Reaktionsgemisch in einen dem Reaktionsteil nachgeschalteten Aufarbeitungsteil der Anlage geführt, in dem eine Aufarbeitung in Produktströme aus Isocyanat, Lösungsmittel und Nebenprodukte erfolgt. Das aufgearbeitete Lösungsmittel wird üblicherweise dem Verfahren wieder zugeführt.

Bedingt durch die Anwesenheit von Phosgen, sowohl im Reaktions- als auch im Aufarbeitungsteil der vorstehend beschriebenen Anlagen, besteht die Gefahr, dass im Falle eines Wassereintritts Phosgen mit Wasser zu CO₂ und Salzsäure reagiert, was bei Anwesenheit von weiterem Wasser zu Korrosionsschäden führen kann. Derartige Korrosionsschäden sind schwer vorhersehbar, führen aber zu lang andauernden Stillständen der Anlagen, was mit hohen Kosten verbunden ist.

Ein Dokument, das sich mit der Detektion von Wassereintritten in Phosgen bzw. Phosgenführenden Anlagen befasst, ist die DD-A 139 955. Dieses Dokument offenbart die Installation von Messeinrichtungen, die das Auftreten von geringen Wasserspuren an so genannten Gefährdungsstellen der Anlage detektieren können. Als Gefährdungsstellen werden dabei insbesondere Wärmetauscher, die durch Spannungsrisskorrosion, Schweißporen oder Materialfehler Undichtigkeiten aufweisen können, definiert.

Messsonden zur Überwachung von Korrosion sind dem Fachmann bekannt. DE-A 28 09 322 offenbart beispielsweise Sonden zur Korrosionsüberwachung in einem Medium. Dabei werden Elektroden aus dem korrosionsgefährdeten Material in einem Sondengehäuse in das korrosive Medium eingebracht. Durch die Elektroden fließt ein ständiger Strom, dessen Stärke vom Maß der Korrodierung der Elektroden abhängt. Die Elektroden sind dabei in bekannter Weise an eine Widerstandsmessbrücke angeschlossen, wobei die Elektroden der Sonde einen Zweig einer Wheatstoneschen Messbrücke bilden. Die anderen Zweige der Messbrücke werden u.a. von einem Überwachungselement und einem Bezugselement gebildet, die jeweils nicht dem korrosiven Medium ausgesetzt sind, sondern von diesem isoliert in einem anderen Teil des Sondengehäuses angeordnet sind.

Aufgabe der vorliegenden Erfindung ist es, ein Verfahren zum Überwachen und Erfassen von Wassereintritten und somit zum Überwachen von Korrosion in Anlagen zur Herstellung von Isocyanaten durch Umsetzung von Phosgen mit einem oder mehreren primären Amin(en) zur Verfügung zu stellen. Eine weitere Aufgabe der vorliegenden Erfindung besteht darin, eine Vorrichtung zur Herstellung von Isocyanaten zur Verfügung zu stellen, welche das Erkennen von Wassereintritten und das daraus resultierende Auftreten von Korrosion ermöglicht.

Gelöst wird die Aufgabe durch ein Verfahren zur Erfassung von Wassereintritten in Anlagen zur Herstellung von Isocyanaten durch Umsetzung von Phosgen mit einem oder mehreren primären Amin(en) in einem Lösungsmittel, wobei diese Anlagen einen Reaktions- und einen Aufarbeitungsteil mit Lösungsmittelrückgewinnung umfassen. Das erfindungsgemäße Verfahren umfasst die Schritte
(a) Einbringen mindestens einer Messsonde in eine kondensierte Phase im Aufarbeitungsteil der Anlage, in der der Gehalt an Isocyanat oder Phosgen unter 5 Vol.%, bevorzugt unter 3 Vol.-%, besonders bevorzugt unter 1 Vol.-% liegt,
(b) Erfassen mindestens eines Signals welches von der mindestens einen Messsonde generiert wird, und
(c) Erfassen von Wassereintritten in den Anlagen durch Überwachen des mindestens einen Signals.

Das erfindungsgemäße Verfahren ermöglicht so erfolgreich die Überwachung von Anlagen zur Herstellung von Isocyanaten und ermöglicht es, auf Wassereintritte sofort zu reagieren, bevor größere Korrosionsschäden an den Anlagen entstehen.

Das Verfahren dient insbesondere der Überwachung von Anlagen, in denen mit wasserfreien Prozessströmen, gearbeitet wird und bei denen beim Eindringen von Wasser, selbst in äußerst kleinen Mengen, beispielsweise im Bereich von etwa 100 ppm, durch Bildung von wässrigen Salzsäuren sich die Korrosivität der Prozessströme stark erhöht. Derartige Anlagen sind Anlagen zur Herstellung von Isocyanaten, Säurechloriden, Polycarbonaten und Säureanhydriden. Bei den Isocyanaten sind die Anlagen bevorzugt diejenigen, in denen mindestens ein Isocyanat aus der Gruppe Methylen-di(phenylisocyanat) (MDI), Toluylendiisocyanat (TDI), Hexamethylendiisocyanat, Pentamethylendiisocyanat (HDI), Isophorondiisocyanat (IPDI), 2,4- und 2,6-Di-isocyanatmethylcyclohexan hergestellt wird.

Erfindungsgemäß werden in Verfahrensschritt (a) eine oder mehrere Messsonde(n) in eine kondensierte Phase im Aufarbeitungsteil der Anlagen, in der der Gehalt an Isocyanat oder Phosgen unter 5 Vol.-%, bevorzugt unter 3 Vol.%, besonders bevorzugt unter 1 Vol.-% liegt eingebracht. Das Einbringen bzw. Anordnen der Messsonde(n) in dem Aufarbeitungsteil der Anlagen in einer kondensierten Phase, in der der Gehalt an Isocyanat oder Phosgen unter 5 Vol.%, bevorzugt unter 3 Vol.%, besonders bevorzugt unter 1 Vol.-% liegt, ermöglicht es, durch Erfassen des mindestens einen Signals welches von der mindestens einen Messsonde generiert wird in Verfahrensschritt (b) und Überwachen des Signals in Verfahrensschritt (c) bereits Spuren von Wasser, die beispielsweise über Leckagen an Dichtelementen, Schweißnähten oder Korrosionsstellen in die Anlagen eintreten können, zu detektieren und korrosive Zustände vorbeugend und frühzeitig zu erkennen. Hierdurch wird die Verfügbarkeit der Anlagen erheblich erhöht, da weniger korrosionsbedingte Stillstände auftreten. Im Rahmen der Erfindung stellte sich nämlich heraus, dass insbesondere im Aufarbeitungsteil dieser Anlagen Korrosion auftritt die so rechtzeitig beobachtet werden kann. Dies hängt wahrscheinlich damit zusammen, dass im Reaktionsteil der Anlagen eine gute Durchmischung und hohe Temperaturen vorherrschen, was die Reaktion von Isocyanat mit eintretendem Wasser begünstigt und beschleunigt, wodurch es in diesem Bereich der Anlagen zu nahezu keiner Korrosion kommen kann.

Die Anordnung von Messsonden an Gefährdungsstellen, wie Wärmetauschern, wie es in der DD 139 955 offenbart wird, ermöglicht es, drastische Wassereinbrüche zu detektieren, doch wird die Anwendung dieser Sonden ausschließlich auf phosgenführende Anlagenkomponenten beschränkt.

Das Einbringen der mindestens einen Messsonde in eine kondensierte Phase im Aufarbeitungsteil der Anlagen, mit einem geringen Gehalt an Phosgen, in der der Gehalt an Isocyanat unter 5 Vol.-%, bevorzugt unter 3 Vol.%, besonders bevorzugt unter 1 Vol.-% liegt, bedeutet im Rahmen der Erfindung, dass die Messsonde bevorzugt an Stellen wie Rohrleitungen, die dem Transport von Flüssigkeitsströmen dienen, Betriebsmittelreservoiren von Vakuumpumpen, an Rohrleitungen am Kopf von Kolonnen, insbesondere am Kopf von Vakuumkolonnen oder an Stellen wie Kondensatoren am Kopf von Destillations- bzw. Rektifikationskolonnen die im Vakuum betrieben werden eingebaut werden. Besonders bevorzugt ist dabei der der Einbau der Messsonden am Kopf von Kolonnen, bevorzugt am Kopf von Vakuumkolonnen, oder den Vakuumkolonnen nachgeschalteten Rohrleitungen oder den Vakuumkolonnen selbst, insbesondere wenn die Vakuumanlage als Flüssigkeitsringverdichter ausgeführt ist.

Unter "Anlagen zur Herstellung von Isocyanaten durch Umsetzung von Phosgen mit einem oder mehreren primären Amin(en) in einem Lösungsmittel" werden im Rahmen der Erfindung Anlagen verstanden, in der die Phosgenierung des Amins oder der Amine in Anwesenheit eines Lösungsmittels oder Lösungsmittelgemisches in einer flüssigen Phase mit Phosgen durchgeführt wird. Gemäß einer weiteren Ausführungsform der Erfindung ist unter "Anlagen zur Herstellung von Isocyanaten durch Umsetzung von Phosgen mit einem oder mehreren primären Amin(en) in einem Lösungsmittel" eine Anlage zu verstehen, in der die Isocyanate durch Umsetzung von Phosgen und Amin in der Gasphase hergestellt werden und anschließend, beispielsweise in einem Quench, in einem Lösungsmittel gelöst werden.

Als Lösungsmittel können allgemein für die Herstellung von Isocyanaten geeignete Lösungsmittel eingesetzt werden. Vorzugsweise sind dies inerte aromatische, aliphatische oder cyclische Kohlenwasserstoffe oder deren halogenierte Derivate. Beispiele für solche Lösungsmittel sind aromatische Verbindungen wie Mono- oder Dichlorbenzol, beispielsweise o-Dichlorbenzol, Toluol, Xylole, Naphthalinderivate wie Tetralin oder Dekalin, Alkane mit etwa 5 bis etwa 12 C-Atomen wie Hexan, Heptan, Octan, Nonan oder Dekan, Cycloalkane wie Cyclohexan, weitgehend inerte Ester und Ether wie Ethyl- oder Butylacetat, Tetrahydrofuran, Dioxan oder Diphenylether. Gemäß einer bevorzugten Ausführungsform ist das Lösungsmittel aus der Gruppe Mono-, Dichlorbenzol, Cyclohexan und Toluol ausgewählt.

Als Amine eignen sich prinzipiell alle primären Amine, die in geeigneter Weise mit Phosgen zu Isocyanaten reagieren können. Geeignet sind dabei alle linearen oder verzweigten, gesättigten oder ungesättigten aliphatischen oder cycloaliphatischen oder aromatischen primären Mono- oder Polyamine, die mit Phosgen zu Isocyanaten umgesetzt werden können. Beispiele für geeignete Amine sind 1,3-Propylendiamin, 1,4-Butylendiamin, 1,5-Pentamethylendiamin, 1,6-Hexamethylendiamin und die entsprechenden höheren Homologen dieser Reihe, Isophorondiamin (IPDA), Cyclohexylendiamin, Cyclohexylamin, Anilin, Phenylendiamin, p-Toluidin, 1,5-Naphthylendiamin, 2,4- oder 2,6-Toluylendiamin oder deren Gemische, 4,4'-, 2,4'- oder 2,2'-Diphenylmethandiamin oder deren Gemische sowie höhermolekulare isomere, oligomere oder polymere Derivate der oben genannten Amine und Polyamine.

Die Umsetzung von Phosgen mit Aminen erfolgt in einem Reaktionsraum im Reaktionsteil der Anlagen, der im Allgemeinen in einem Reaktor angeordnet ist, d.h. unter Reaktionsraum wird der Raum verstanden, in dem ein für die Ausbeute des Verfahrens relevanter Teil der Umsetzung der Edukte und/oder Zwischenprodukte erfolgt, und somit das Isocyanat gebildet wird. Unter Reaktor wird die technische Vorrichtung verstanden, die den Reaktionsraum enthält. Hierbei kann es sich um alle üblichen aus dem Stand der Technik bekannten Reaktionsräume handeln, die zur Darstellung von Isocyanaten geeignet sind. Gemäß einer Ausführungsform der Erfindung ist dem Reaktionsteil eine Mischeinrichtung vorgeschaltet, in der die Edukte, das Phosgen und das mindestens eine primäre Amin sowie das Lösungsmittel, das auch in Form eines Gemisches mit Phosgen und/oder Amin der Reaktion zugeführt werden kann, gemischt und anschließend dem Reaktionsteil zugeführt werden. Bei der Mischeinheit kann es sich beispielsweise um eine Mischdüse handeln.

Nachdem die Edukte im Reaktionsteil der Anlagen umgesetzt wurden, führt man das erhaltene Reaktionsgemisch in den Aufarbeitungsteil der Anlage.

Unter dem Begriff "Aufarbeitungsteil" wird im Sinne der vorliegenden Erfindung der Teil der Anlagen verstanden, in dem eine oder mehrere kolonnenähnlichen Systeme zur thermischen Trennung von Isocyanat, Lösungsmittel und gegebenenfalls Nebenprodukt angeordnet sind. Das Reaktionsgemisch, das im Wesentlichen aus den Isocyanaten, dem Lösungsmittel, Salzsäure und Phosgen besteht, wird in diesem Teil der jeweiligen Anlage mittels Destillation und/oder Rektifikation, sowie gegebenenfalls thermischer Rückstandsbehandlung in Knetern oder Schaufeltrocknern in seine Bestandteile aufgetrennt, wobei das Lösungsmittel dem Reaktionsteil der Anlage wieder zugeführt werden kann. Im Rahmen der Erfindung wurde dabei beobachtet, dass durch das Einbringen bzw. Anordnen von Messsonden im Aufarbeitungsteil der Anlage, das Auftreten von korrosiven Mediumsbedingungen in den Anlagen besonders gut detektiert und somit das Auftreten von Korrosion wirksam verhindert werden kann.

Unter dem Begriff "Messsonde" werden im Rahmen der Erfindung Elektroden bzw. Sensoren bzw. Bauteile verstanden, die ein Signal liefern können, welches ein Maß für Korrosion ist. Derartige Messsonden sind dem Fachmann beispielsweise aus DE-A 28 09 322 und DD-A 139 955 bekannt. Gemäß einer Ausführungsform der Erfindung hat es dabei sich als vorteilhaft herausgestellt, in Verfahrensschritt (a) eine Messsonde einzubringen, die einen Widerstandsdraht umfasst und insbesondere auf dem Prinzip der elektrischen Widerstandsmessung basiert.

Der Widerstandsdraht umfasst dabei Werkstoffe, die einerseits eine geringere Korrosionsbeständigkeit aufweisen als die Werkstoffe der Apparate und Rohrleitungen der Anlage, andererseits aber bei bestimmungsgemäßem Betrieb der Anlage eine so hohe Korrosionsbeständigkeit aufweisen, dass die Funktion der Sonde über mehrere Monate hinweg gewährleistet ist. Dafür geeignete Werkstoffe sind Metalle aus der Gruppe Zn und Fe, sowie niedrig oder auch hoch legierte Stähle sowie dem Fachmann bekannte Nickelbasislegierungen.

Im Falle eines Wassereintritts erfolgt bei geringen vorhandenen HCl-Konzentrationen die Bildung von wässriger Salzsäure in den Anlagen. Die so entstandenen erhöhten korrosiven Mediumsbedingungen greifen bevorzugt den weniger korrosionsbeständigen Sondendraht und weniger die Apparate und Rohrleitungen der Anlage selbst an, was zu einer Verringerung des Querschnittes des Drahtes und somit zu einer Abnahme des Stromflusses und einem Anstieg des elektrischen Widerstandes führt. Dieser Anstieg wird mit dem Fachmann bekannten Methoden, beispielsweise mittels einer Wheatstonesche Brücke gemessen. Während des Betriebs der Vorrichtung wird das Signal bzw. werden die Signale, die von den Messsonden geliefert werden in Verfahrensschritt (b) permanent erfasst und überwacht, um mögliche Wassereintritte in die Vorrichtung detektieren zu können. Lässt der Verlauf des Signals auf Wassereinbrüche schließen, kann eine sofortige Abschaltung bzw. Reparatur der Anlage vorgenommen werden, bevor Schäden durch Korrosion entstehen.

### Die Erfindung wird anhand der nachfolgenden Beispiele näher erläutert:

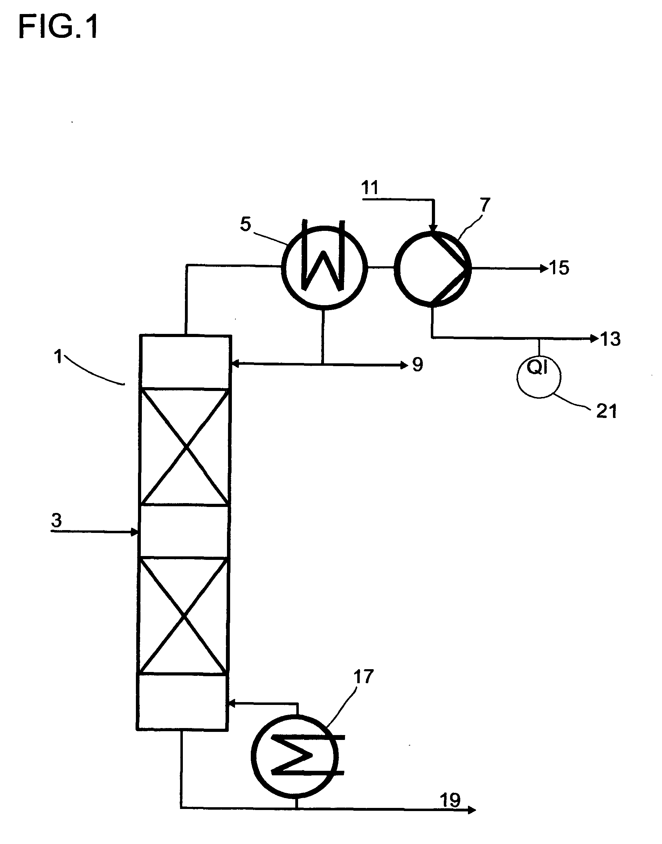

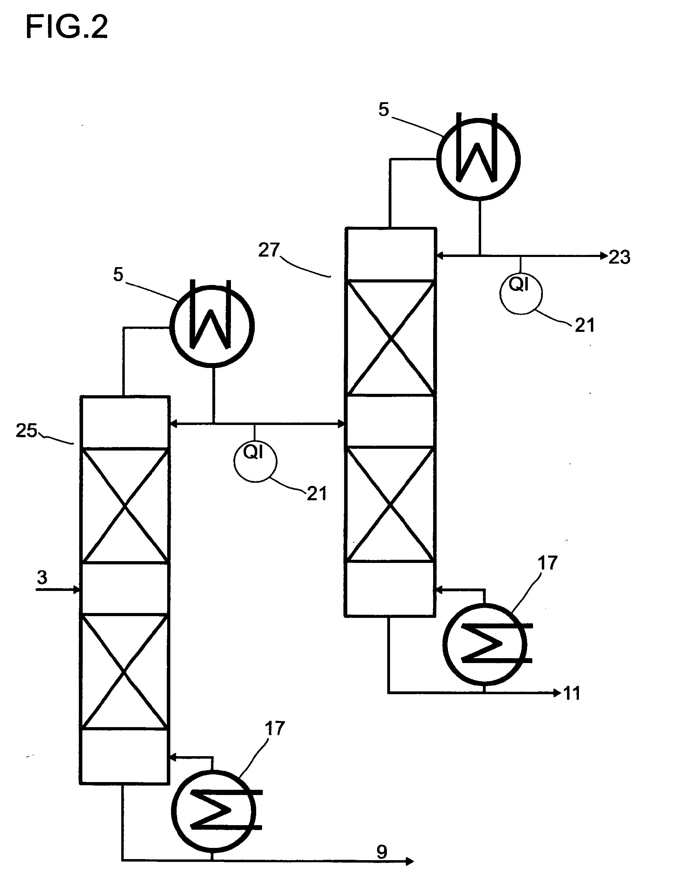

Bevorzugte Ausführungsformen des erfindungsgemäßen Verfahrens sind in den Figuren 1 und 2 schematisch veranschaulicht.

### Beispiel 1

In Figur 1 ist beispielhaft die Abtrennung eines Isocyanatstroms 3 von Schwersiedern 19 und gleichzeitiger Gewinnung des reinen Isocyanats 9 dargestellt. Die dafür eingesetzte Destillationskolonne 1 mit Verdampfer 17 wird im Vakuum betrieben. Während das Isocyanat 9 im Kondensator 5 niedergeschlagen wird, gelangen die leichtsiedenden Komponenten wie HCl, Phosgen oder bei der Synthese auftretender Nebenkomponenten und die Leckluft in die Vakuumpumpe 7. Zur Vakuumerzeugung wird eine Flüssigkeitsringpumpe 7 eingesetzt. Als Betriebsmittel 11 für die Flüssigkeitsringpumpe 7 kommt das im Isocyanatverfahren eingesetzte Lösungsmittel, beispielsweise Monochlorbenzol oder Toluol zum Einsatz.

Bei der Destillation freigesetzte Spuren von Chlorwasserstoff, werden von dem Betriebsmittel 11 der Vakuumpumpe 7 mit Abluftstrom 15 teilweise absorbiert und reichern sich in Strom 13 an. Das aufgrund üblicher technischer Leckraten und Leckluft in Form von Luftfeuchtigkeit in das System eindringende Wasser wird ebenfalls absorbiert und angereichert. Durch die erfindungsgemäße, gezielte Platzierung der Messsonde 21 in dem Aufarbeitungsteil der Anlage, in diesem Fall in Strom 13, lässt sich der Eintritt von Wasser gezielt überwachen.

Die sich einstellende Konzentration an Chlorwasserstoff und Wasser hängen während des Betriebs der Anlage vom, zu- und abgeführten Lösungsmittelstrom ab und sind im Normalbetrieb zu gering, um Korrosion zu verursachen. Die Messsonde 21 zeigt während des Betriebes der Anlage einen zwar schwankenden, insgesamt aber auf konstantem Niveau verlaufenden, für den Einbauort charakteristischen Wert an.

Kommt es zum Eintritt von Wasser in das System, beispielsweise durch Leckagen in mit Dampf oder Kühlwasser betriebenen Wärmetauschern oder erhöhten Leckraten in den Vakuumpumpen 7, so erhöht sich der Wassergehalt und mit dem anwesenden Chlorwasserstoff die Korrosivität des Betriebsmittels. Entsprechend steigt der Messwert der eingebauten Messsonde 21 an.

Die Messung der erhöhten Korrosionsrate erfolgt dabei so sensibel, dass ausreichend Zeit zur Fehlersuche oder Gegenmaßnahmen bleibt.

### Beispiel 2

Figur 2 zeigt beispielhaft einen Ausschnitt der destillativen Aufbereitung von Produktströmen 3 im Aufarbeitungsteil von Isocyanatanlagen.

In der ersten Destillationskolonne 25 mit Wärmetauscher 17 und Kondensator 5 wird das Lösungsmittel 11, zum Beispiel Monochlorbenzol oder Toluol, über Kopf abgetrennt. Das so erhaltene Lösungsmittel 11 enthält noch Spuren anderer im Prozess auftretender leichtsiedender Stoffe wie HCl, Phosgen oder bei der Synthese auftretender Nebenkomponenten. Dieses Lösungsmittel 11 kann nicht ohne weitere Behandlung im Prozess wieder eingesetzt werden. In einer zweiten Destillationskolonne 27 Wärmetauscher 17 und Kondensator 5 wird das Lösungsmittel 11 dadurch gereinigt, dass die Leichtsieder 23 mit einem gewissen Anteil des Lösungsmittels 11 über Kopf abgetrennt und an anderer Stelle im Prozess weiter behandelt werden.

Im störungsfreien Betrieb zeigen die Messsonden 21 einen sehr niedrigen, konstanten Messwert an.

Gelangt durch eine Störung wie zum Beispiel Leckage in einem der mit Dampf oder Wasser betriebenen Wärmetauscher der ersten Destillationskolonne 17 Wasser in das System, so wird das Wasser mit dem Lösungsmittel 11 in den Kopf der Kolonne 25 gelangen. Mit dem dort ebenfalls anwesenden Chlorwasserstoff, HCl, entsteht ein korrosives Lösungsmittelgemisch. Dies wird von der Messsonde 21 erkannt und führt zu einem Anstieg des Signals.

Weiterhin gelangt das korrosive Lösungsmittelgemisch in die zweite Destillationskolonne 27. Die gegenüber dem Lösungsmittel 11 leichtsiedenden Stoffe Wasser und Chlorwasserstoff werden über Kopf abgetrennt und bilden wieder ein entsprechend korrosives Kondensat. Dies wird von der Messsonde 21, die am Kopf der zweiten Destillationskolonne angeordnet ist, zeitlich verzögert erkannt.

Bei einem flächendeckenden Einsatz der Messsonden im Prozess lässt sich somit die Eintrittsstelle des Wassers lokalisieren und die Verbreitung im Prozess nachvollziehen.

## Patentansprüche

1. Verfahren zur Erfassung von Wassereintritten in Anlagen zur Herstellung von Isocyanaten durch Umsetzung von Phosgen mit einem oder mehreren primären Amin(en) in einem Lösungsmittel, wobei diese Anlagen einen Reaktions- und einen Aufarbeitungsteil mit Lösungsmittelrückgewinnung umfassen, umfassend die Schritte
(a) Einbringen mindestens einer Messsonde in eine kondensierte Phase im Aufarbeitungsteil der Anlage, in der der Gehalt an Isocyanat oder Phosgen unter 5 Vol.-% liegt,
(b) Erfassen mindestens eines Signals welches von der mindestens einen Messsonde generiert wird, und
(c) Erfassen von Wassereintritten in den Anlagen durch Überwachen des mindestens einen Signals.

2. Verfahren nach Anspruch 1, wobei in Verfahrensschritt (a) eine oder mehrere Messsonde(n) eingebracht wird bzw. werden, die einen Widerstandsdraht umfasst bzw. umfassen.

3. Verfahren nach Anspruch 1 oder 2, wobei in Verfahrensschritt (b) das Signal in Form des Stromflusses durch die Messsonde(n) erfasst wird.

4. Verfahren nach einem der Ansprüche 1 bis 3, wobei in dem Aufarbeitungsteil der Anlagen das Lösungsmittel von dem hergestellten Isocyanat abgetrennt und in den Reaktionsteil zurückgeführt wird.

5. Verfahren nach einem der Ansprüche 1 bis 4, wobei in dem Aufarbeitungsteil der Anlagen das Lösungsmittel von dem hergestellten Isocyanat in einem oder mehreren kolonnenähnlichen System(en) abgetrennt wird.

6. Verfahren nach einem der Ansprüche 1 bis 5, wobei die Abtrennung des Lösungsmittels von dem hergestellten Isocyanat mittels eines oder mehrerer Trennverfahren(s) ausgewählt aus Destillation, Rektifikation und Extraktion durchgeführt wird.

7. Verfahren nach einem der Ansprüche 1 bis 6, wobei die eine oder mehreren Messsonde(n) an einer mit Lösungsmittel betriebenen Vakuumpumpe eingebracht wird bzw. werden.

8. Verfahren nach einem der Ansprüche 1 bis 7, wobei als Isocyanat mindestens ein Isocyanat aus der Gruppe Methylen-di(phenylisocyanat), Toluylendiisocyanat, Hexamethylendiisocyanat, Pentamethylendiisocyanat, Isophorondiisocyanat, 2,4- und 2,6-Diisocyanatmethylcyclohexan hergestellt wird.

9. Verfahren nach einem der Ansprüche 1 bis 8, wobei das Lösungsmittel aus der Gruppe Mono-, Dichlorbenzol, Cyclohexan und Toluol ausgewählt ist.

10. Vorrichtung zur Durchführung eines Verfahrens nach einem der Ansprüche 1 bis 9, **dadurch gekennzeichnet, dass** die Messsonde (21) in die kondensierte Phaseim Aufarbeitungsteil der Anlage eingesetzt wird.

## Claims

1. A method of measuring entry of water into plants for preparing isocyanates by reacting phosgene with one or more primary amine(s) in a solvent, with these plants comprising a reaction section and a work-up section with solvent recovery, which comprises the steps
(a) introduction of at least one measurement probe into a condensed phase in the work-up section of the plant in which the content of isocyanate or phosgene is below 5% by volume,
(b)measurement of at least one signal which is generated by the at least one measurement probe and
(c) measurement of entry of water into the plants by monitoring the at least one signal.

2. The method according to claim 1, wherein one or more measurement probe(s) which comprises or comprise a resistance wire is/are introduced in step (a).

3. The method according to claim 1 or 2, wherein the signal is measured in the form of the flow of current through the measurement probe(s) in step (b).

4. The method according to any of claims 1 to 3, wherein, in the work-up section of the plants, the solvent is separated off from the product isocyanate and recirculated to the reaction section.

5. The method according to any of claims 1 to 4, wherein, in the work-up section of the plants, the solvent is separated off from the product isocyanate in one or more column-like system(s).

6. The method according to any of claims 1 to 5, wherein the separation of the solvent from the product isocyanate is carried out by means of one or more separation process(es) selected from among distillation, rectification and extraction.

7. The method according to any of claims 1 to 6, wherein the one or more measurement probe(s) is/are installed at a vacuum pump operated using solvent.

8. The method according to any of claims 1 to 7, wherein at least one isocyanate from the group consisting of methylenedi(phenyl isocyanate), tolylene diisocyanate, hexamethylene diisocyanate, pentamethylene diisocyanate, isophorone diisocyanate, 2,4- and 2,6-diisocyanatomethylcyclohexane is prepared as isocyanate.

9. The method according to any of claims 1 to 8, wherein the solvent is selected from the group consisting of monochlorobenzene, dichlorobenzene, cyclohexane and toluene.

10. An apparatus for carrying out a method according to any of claims 1 to 9, wherein the measurement probe (21) is inserted into the condensed phase in the work-up section of the plant.

## Revendications

1. Procédé pour la détection d'entrées d'eau dans des installations dédiées à la production d'isocyanates par mise en réaction de phosgène avec une ou plusieurs aminé(s) primaire(s) dans un solvant, ces installations comprenant une section réaction et une section traitement final avec récupération de solvant, comprenant les étapes
(a) introduction d'au moins une sonde de mesure dans une phase condensée dans la section traitement final de l'installation, dans laquelle la teneur en isocyanate ou phosgène est inférieure à 5 % en volume,
(b) détection d'au moins un signal qui est engendré par ladite au moins une sonde de mesure, et
(c) détection d'entrées d'eau dans les installations par surveillance dudit au moins un signal.

2. Procédé selon la revendication 1, dans lequel on introduit dans l'étape (a) du procédé une ou plusieurs sonde(s) de mesure qui comprend/comprennent un fil de résistance.

3. Procédé selon la revendication 1 ou 2, dans lequel on détecte par la/les sonde(s) de mesure dans l'étape (b) du procédé le signal sous forme du flux de courant.

4. Procédé selon l'une quelconque des revendications 1 à 3, dans lequel dans la section traitement final des installations le solvant est séparé de l'isocyanate produit et recyclé dans la section réaction.

5. Procédé selon l'une quelconque des revendications 1 à 4, dans lequel dans la section traitement final des installations le solvant est séparé de l'isocyanate produit, dans un ou plusieurs système(s) de type colonne.

6. Procédé selon l'une quelconque des revendications 1 à 5, dans lequel la séparation du solvant d'avec l'isocyanate produit est effectuée au moyen d'un ou plusieurs procédé(s) de séparation choisi(s) parmi la distillation, la rectification et l'extraction.

7. Procédé selon l'une quelconque des revendications 1 à 6, dans lequel ladite/lesdites une ou plusieurs sonde(s) de mesure est/sont introduite(s) sur une pompe à vide fonctionnant avec un solvant.

8. Procédé selon l'une quelconque des revendications 1 à 7, dans lequel en tant qu'isocyanate est produit au moins un isocyanate choisi dans le groupe constitué par le méthylène-di(phénylisocyanate), le toluylène-diisocyanate, l'hexaméthylène-diisocyanate, le pentaméthylène-diisocyanate, l'isophorone-diisocyanate, le 2,4- et le 2,6-diisocyanate-méthylcyclohexane.

9. Procédé selon l'une quelconque des revendications 1 à 8, dans lequel le solvant est choisi dans le groupe constitué par le monochlorobenzène, le dichlorobenzène, le cyclohexane et le toluène.

10. Dispositif pour la mise en oeuvre d'un Procédé selon l'une quelconque des revendications 1 à 9, **caractérisé en ce que** la sonde de mesure (21) est insérée dans la phase condensée dans la section traitement final de l'installation.
